# EUROPEAN PATENT SPECIFICATION

(11) **EP 3 002 276 B1**
(45) Date of publication and mention of the grant of the patent: **13.12.2017**
(21) Application number: 13871854.9
(22) Date of filing: 25.06.2013
(51) Int. Cl.: C07C 251/46, C07C 249/08, C07D 213/79, C07D 213/803, C07D 333/38, A61P 35/00, A61P 35/02, A61K 31/4402, A61K 31/381, A61K 31/222, A61K 31/235, A61K 31/15

(54) **SHIKONIN, ALKANNIN, AND RACEMIC PARENT NUCLEUS CARBONYL OXIME DERIVATIVES AND APPLICATIONS THEREOF**
SHIKONIN, ALKANNIN UND RACEMISCHE MUTTERKERN-CARBONYLOXIMDERIVATE SOWIE ANWENDUNGEN DAVON
SHIKONINE, ALCANNINE, ET DÉRIVÉS CARBONYLOXIMES DE NOYAU PARENT RACÉMIQUE ET APPLICATIONS DE CEUX-CI

(30) Priority: 16.01.2013 CN 201310016436; 04.02.2013 CN 201310044118
(43) Date of publication of application: 06.04.2016
(73) Proprietor: Shanghai Jiao Tong University, Shanghai 200240 (CN)
(72) Inventor: LI, Shaoshun, Shanghai 200240 (CN); WANG, Rubing, Shanghai 200240 (CN); ZHENG, Xiaogang, Shanghai 200240 (CN); ZHANG, Xu, Shanghai 200240 (CN)
(74) Representative: Sun, Yiming
(86) International application number: PCT/CN2013/077849
(87) International publication number: WO 2014/110889

(56) References cited:
- WO-A1-2009/068322
- CN-A- 1 112 363
- CN-A- 1 420 111
- CN-A- 101 265 212
- CN-A- 101 863 786
- CN-A- 102 399 139
- CN-A- 102 557 914
- CN-A- 102 557 914
- CN-A- 102 617 342
- CN-A- 103 130 680
- CN-A- 103 145 583
- WEN ZHOU ET AL: "Semi-synthesis and antitumor activity of 6-isomers of 5, 8--dimethyl acylshikonin derivatives", EUROPEAN JOURNAL OF MEDICINAL CHEMISTRY, EDITIONS SCIENTIFIQUE ELSEVIER, PARIS, FR, vol. 46, no. 8, 4 May 2011 (2011-05-04), pages 3420-3427, XP028377964, ISSN: 0223-5234, DOI: 10.1016/J.EJMECH.2011.05.006 [retrieved on 2011-05-12]
- XU, CHAOHUI: 'Preparation of 5,8-dimethoxyl-1,4-naphthoquinone-6-formic acid derivatives and studies on their antitumor activities' MASTER'S DISSERTATION OF SHANGHAI JIAO TONG UNIVERSITY 2007, XP008179443
- ZHANG, LIHONG: 'Theoretical Study on Antitumor Activities of Naphthoquinone Derivatives' MASTER'S DISSERTATION OF NORTHEAST NORMAL UNIVERSITY 2012, XP055257201

## Description

### BACKGROUND OF THE PRESENT INVENTION

### Field of Invention

The present invention relates to a series of derivatives of shikonin, alkannin and a racemic mixture thereof with hydroxyl methylation and carbonyl oximation on the naphthazarin parent nucleus, and their application in the medicine field.

### Description of Related Arts

Purple gromwell is a traditional Chinese medicine commonly used in clinical, said medicine being included in the Pharmacopoeia of the People's Republic of China. There are two types of purple gromwell, i.e., *Lithospermum erythrorhizon* (or named as northeast purple gromwell) and *A. euchroma Johnst* (or named as Xinjiang purple gromwell). The main effective component of *Lithospermum erythrorhizon* is shikonin and its derivatives, while *A. euchroma Johnst* comprises alkannin and its derivatives; shikonin (R-isomer) and alkannin (S-isomer) being a pair of enantiomers. Shikonin and alkannin have been proved to have multiple biological activities including anti-inflammatory, promoting wound healing, antibacterial, antiviral, antithrombotic, antithyroid, immunoregulatory, hypoglycemic, hepatoprotective activities. In recent years, there has been increasing research interest in the antitumor activities of shikonin and alkannin and their derivatives, the research of using said compounds as lead compounds has become the focus of its technical filed.

The structural modifications made to the derivatives of shikonin and alkannin as reported in the prior art mainly focused on the side chain and the naphthazarin ring, i.e., (1) in one case, the structure of the naphthazarin ring (5,8-dihydroxy-1,4-naphthoquinone) was kept while the hydroxyl group in the side chain was modified (CN142011, CN1112363), (2) in another case, modification of the naphthazarin ring was performed, including alkylation or acetylation, obtaining the corresponding shikonin and alkannin derivatives (Chinese patent application number: 201010046435.2, 201010209926.3, 201210021929.3 and 201010065488.7). Additionally, Zhou et al. reported on semi-synthesis and antitumor activity of 6-isomers of 5,8-dimethyl acylshikonin derivatives (see Europ J Med Chemistry 2011, 46(8), 3420-2437). CN 103130680, CN 103145583, and CN 102557914 provide further examples of ashikonin derivatives demonstrating anticancer activity.

### SUMMARY OF THE PRESENT INVENTION

An object of the present invention is to provide derivatives of shikonin, alkannin and a racemic mixture thereof with hydroxyl methylation and carbonyl oximation on the naphthazarin parent nucleus, as well as applications thereof. The compounds of the present invention are novel in structure, easy to prepare and have good inhibitory activity against tumor cells in vitro.

The present invention is realized by the technical solution in the following:
The present invention involves derivatives of shikonin, alkannin and a racemic mixture thereof with hydroxyl methylation and carbonyl oximation on the naphthazarin parent nucleus;
wherein the derivatives of shikonin with hydroxyl methylation and carbonyl oximation on the naphthazarin parent nucleus have structural formula (I) or (II): 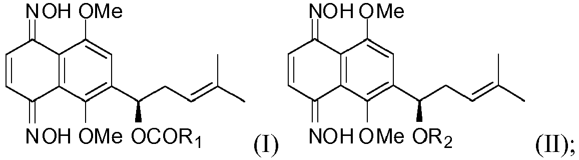 the derivatives of alkannin with hydroxyl methylation and carbonyl oximation on the naphthazarin parent nucleus have structural formula (III) or (IV): 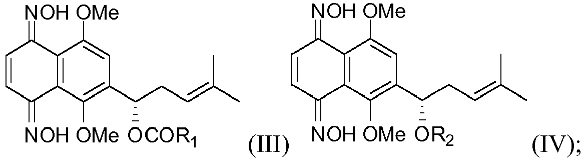 the racemic mixture of derivatives of shikonin and alkannin with hydroxyl methylation and carbonyl oximation on the naphthazarin parent nucleus have structural formula (V) or (VI): 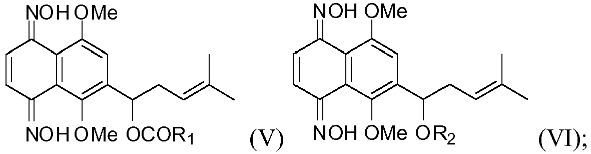 wherein, R₁ is C₁-C₆ alkane, C₁-C₆ olefin, C₁-C₆ arene, or substituted C₁-C₆ arene; and R₂ is C₁-C₆ alkane, C₁-C₆ olefin, C₁-C₆ arene or substituted C₁-C₆ arene, or is H.

Preferably, R₁ in the above derivatives of shikonin, alkannin and a racemic mixture thereof with hydroxyl methylation and carbonyl oximation on the naphthazarin parent nucleus is methyl, isopropyl, isobutyl, 2-hydroxyl-2-methylpropyl, phenyl, 2-fluorophenyl, 4-fluorophenyl, 1-methylethylene, 2-clorophenyl, 4-clorophenyl, 4-methoxyphenyl, ethylene, 2-thiophenyl, 4-nitrophenyl or 2-pyridyl; R₂ is hydrogen, methyl, ethyl or isopentyl.

A preparation method of above derivatives of shikonin, alkannin and a racemic mixture thereof with hydroxyl methylation and carbonyl oximation on the naphthazarin parent nucleus is described herein. For the derivatives of shikonin with hydroxyl methylation and carbonyl oximation on the naphthazarin parent nucleus having structural formula (I) or (II), the preparation method comprises following steps:
(A) using 1,4,5,8-tetramethoxyshikonin as a starting material, using 4-dimethylaminopyridine (DMAP) as a catalyst, in the presence of N,N'-dicyclohexylcarbodiimide (DCC), the hydroxyl group on the side chain of 1,4,5,8-tetramethoxyshikonin reacts with a carboxylic acid, obtaining ester derivatives;
   or using N,N-Dimethylformamide (DMF) as a solvent, in the presence of NaH, the hydroxyl group on the side chain of 1,4,5,8-tetramethoxyshikonin reacts with alkyl halides, obtaining ether derivatives; and
(B) oxidizing and dimethylating said ester derivatives or ether derivatives by ceric ammonium nitrate (CAN), obtaining dimethyl dicarbonyl compounds, which then couple with hydroxylamine hydrochloride in the presence of anhydrous pyridine, obtaining ester derivatives or ether derivatives of shikonin with hydroxyl methylation and carbonyl oximation on the naphthazarin parent nucleus.

Preferably, in step (A), the molar ratio of 1,4,5,8-tetramethoxyshikonin, DCC and carboxylic acid is 1:1.5:1.2-1:5:2; the molar ratio of 1,4,5,8-tetramethoxyshikonin, NaH and alkyl halide is 1:3:1.5-1:6:4; in step (B), the molar ratio of ester derivatives or ether derivatives and ceric ammonium nitrate is 1:5-1:10, the molar ratio of the oxidized product of ester derivatives or ether derivatives, hydroxylamine hydrochloride and anhydrous pyridine is 1:2.2:3-1:5:8.

A further preparation method of above derivatives of shikonin, alkannin and a racemic mixture thereof with hydroxyl methylation and carbonyl oximation on the naphthazarin parent nucleus is disclosed herein. For the derivatives of alkannin with hydroxyl methylation and carbonyl oximation on the naphthazarin parent nucleus having structural formula (III) or (IV), the preparation method comprises following steps:
(A) using 1,4,5,8-tetramethoxyalkannin as a starting material, using DMAP as a catalyst, in the presence of DCC, the hydroxyl group on the side chain of 1,4,5,8-tetramethoxyalkannin reacts with carboxylic acid, obtaining ester derivatives;
   or using DMF as a solvent, in the presence of NaH, the hydroxyl group on the side chain of 1,4,5,8-tetramethoxyalkannin reacts with alkyl halides, obtaining ether derivatives; and
(B) oxidizing and dimethylating said ester derivatives or ether derivatives by ceric ammonium nitrate, obtaining dimethyl dicarbonyl compounds, which then couple with hydroxylamine hydrochloride in the presence of anhydrous pyridine, obtaining ester derivatives or ether derivatives of alkannin with hydroxyl methylation and carbonyl oximation on the naphthazarin parent nucleus.

Preferably, in step (A), the molar ratio of 1,4,5,8-tetramethoxyalkannin, DCC and carboxylic acid is 1:1.5:1.2-1:5:2; the molar ratio of 1,4,5,8-tetramethoxyalkannin, NaH and alkyl halide is 1:3:1.5-1:6:4; in step (B), the molar ratio of ester derivatives or ether derivatives and ceric ammonium nitrate is 1:5-1:10, the molar ratio of the oxidized product of ester derivatives or ether derivatives, hydroxylamine hydrochloride and anhydrous pyridine is 1:2.2:3-1:5:8.

A further preparation method of above derivatives of shikonin, alkannin and a racemic mixture thereof with hydroxyl methylation and carbonyl oximation on the naphthazarin parent nucleus is disclosed herein. For the derivatives of a racemic mixture of shikonin and alkannin with hydroxyl methylation and carbonyl oximation on the naphthazarin parent nucleus having structural formula (V) or (VI), the preparation method comprises following steps:
(A) using (±)-1,4,5,8-tetramethoxyshikonin as a starting material, using DMAP as a catalyst, in the presence of DCC, the hydroxyl group on the side chain of (±)-1,4,5,8-tetramethoxyshikonin reacts with carboxylic acid, obtaining ester derivatives;
   or using DMF as a solvent, in the presence of NaH, the hydroxyl group on the side chain of (±)-1,4,5,8-tetramethoxyshikonin reacts with alkyl halides, obtaining ether derivatives; and
(B) oxidizing and demethylating said ester derivatives or ether derivatives by ceric ammonium nitrate, obtaining dimethyl dicarbonyl compounds, which then couple with hydroxylamine hydrochloride in the presence of anhydrous pyridine, obtaining ester derivatives or ether derivatives of a racemic mixture of shikonin and alkannin with hydroxyl methylation and carbonyl oximation on the naphthazarin parent nucleus.

Preferably, in step (A), the molar ratio of (±)-1,4,5,8-tetramethoxyshikonin, DCC and carboxylic acid is 1:1.5:1.2-1:5:2; the molar ratio of (±)-1,4,5,8-tetramethoxyshikonin, NaH and alkyl halide is 1:3:1.5-1:6:4; in the step (B), the molar ratio of ester derivatives or ether derivatives and ceric ammonium nitrate is 1:5-1:10, the molar ratio of the oxidized product of ester derivatives or ether derivatives, hydroxylamine hydrochloride and anhydrous pyridine is 1:2.2:3-1:5:8.

The present invention also involves the application of above derivatives of shikonin, alkannin and a racemic mixture thereof with hydroxyl methylation and carbonyl oximation on the naphthazarin parent nucleus in the preparation of antitumor drugs.

Compared with the prior art, the present invention has following advantages:
1. The present invention performs hydroxyl methylation and carbonyl oximation on the naphthazarin parent nucleus of shikonin, alkannin and racemic mixture thereof, obtaining a series of novel derivatives of shikonin, alkannin and a racemic mixture thereof with hydroxyl methylation and carbonyl oximation on the naphthazarin parent nucleus. The starting materials used in the present invention (1,4,5,8-tetramethoxyshikonin and 1,4,5,8-tetramethoxyalkannin with a high chiral purity, >99% ee) were prepared by the method of a disclosed patent application (CN102399139A) of present inventors.
2. The key starting material, (±)-1,4,5,8-tetramethoxyshikonin, used in the present invention was prepared by a single-step reaction following the method in ZL200510025243.1. The starting racemic mixture is easier to obtain and cheap in cost.
3. The derivatives of shikonin, alkannin and a racemic mixture thereof with hydroxyl methylation and carbonyl oximation on the naphthazarin parent nucleus obtained in the present invention show good tumor cell growth inhibitory activities in vitro, and with a selectivity therein, which indicate their promising applications in tumor treatment.

### BRIEF DESCRIPTION OF THE DRAWINGS

The further features, objects and advantages of the present invention will become more apparent in view of the following detailed description of the embodiments with respect to the accompanying drawings.
Figure 1 illustrates a synthetic process of an ester derivative of shikonin with hydroxyl methylation and carbonyl oximation on the naphthazarin parent nucleus (formula I).
Figure 2 illustrates a synthetic process of an ester derivative of alkannin with hydroxyl methylation and carbonyl oximation on the naphthazarin parent nucleus (formula III).
Figure 3 illustrates a synthetic process of an ether derivative of shikonin with hydroxyl methylation and carbonyl oximation on the naphthazarin parent nucleus (formula II).
Figure 4 illustrates a synthetic process of an ether derivative of alkannin with hydroxyl methylation and carbonyl oximation on the naphthazarin parent nucleus (formula IV).
Figure 5 illustrates a synthetic process of 1,4,5,8-tetramethoxyshikonin.
Figure 6 illustrates a synthetic process of 1,4,5,8-tetramethoxyalkannin.
Figure 7 illustrates a synthetic process of an ester derivative of a racemic mixture of shikonin and alkannin with hydroxyl methylation and carbonyl oximation on the naphthazarin parent nucleus (formula V) .
Figure 8 illustrates a synthetic process of an ether derivative of a racemic mixture of shikonin and alkannin with hydroxyl methylation and carbonyl oximation on the naphthazarin parent nucleus (formula VI).
Figure 9 illustrates a synthetic process of (±)-1,4,5,8-tetramethoxyshikonin.

### DETAILED DESCRIPTION OF THE EMBODIMENT

The present invention is described in detail in view of the drawings and embodiments. The following embodiments will help the skilled person understand the present invention, but will not limit the present invention in any way. It is noted that, to the skilled person, the present invention may be further modified and improved within the concept of the present invention. The experimental details which are not specified in the following embodiments are performed according to conventional conditions, or according to the conditions suggested by the producer.

### Embodiment 1

This embodiment involves a series of ester or ether derivatives of shikonin with hydroxyl methylation and carbonyl oximation on the naphthazarin parent nucleus and ester or ether derivatives of alkannin with hydroxyl methylation and carbonyl oximation on the naphthazarin parent nucleus, which has structural formula (I), (II), (III) or (IV): 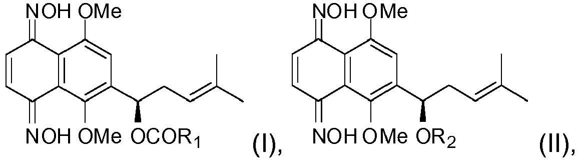 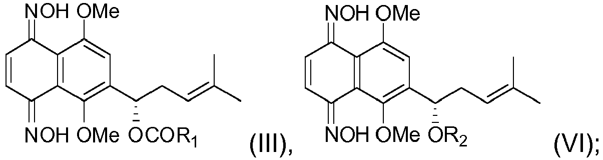 wherein R₁ and R₂ are defined in Table 1.

### 1. Preparation of ester derivatives in series I and III:

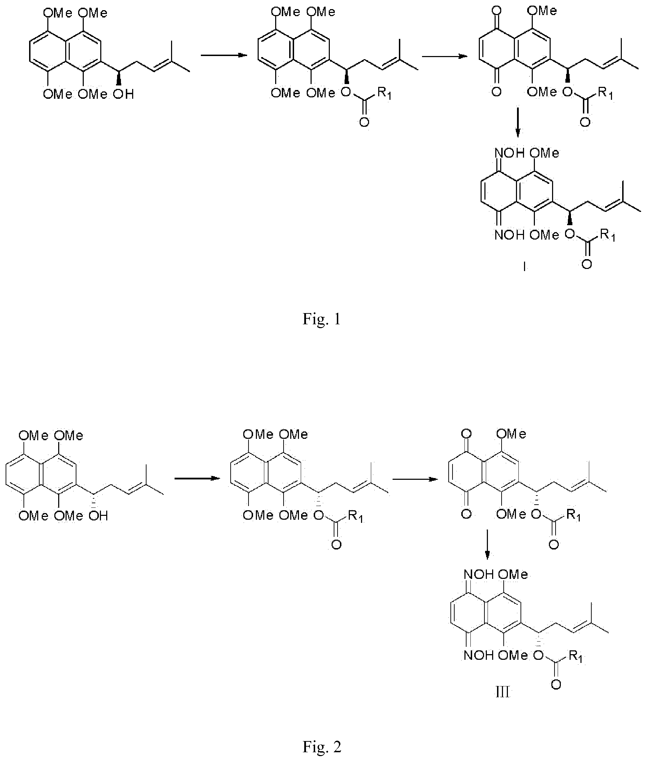

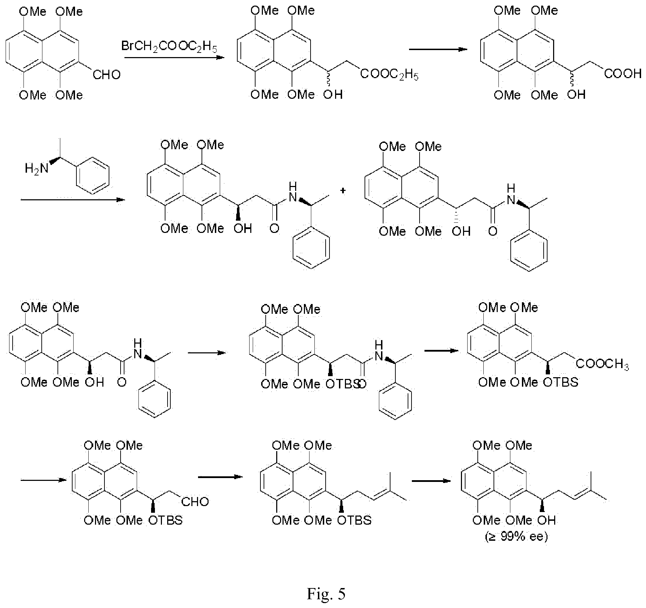

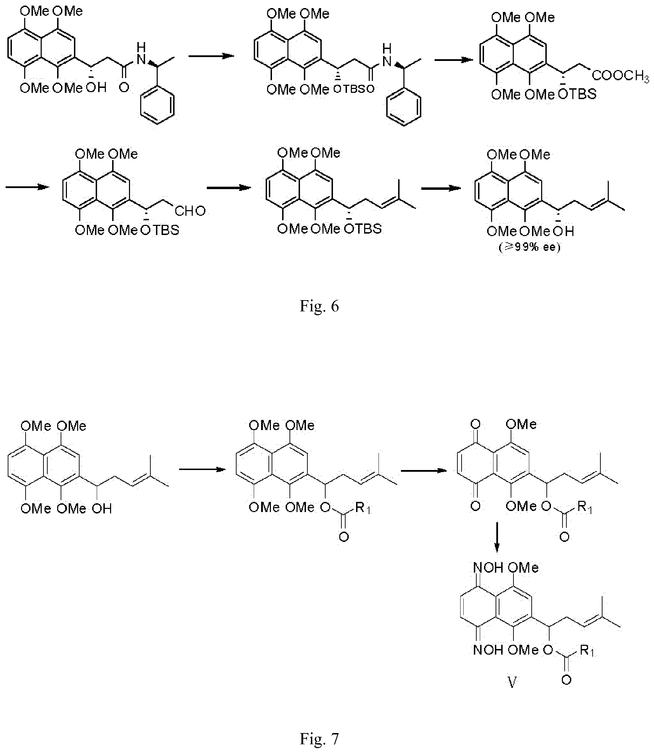

The synthetic route for the ester derivative in series I is shown in Figure 1 and the synthetic route for the ester derivative in series III is shown in Figure 2. The general synthetic methods for the ester derivatives in series I and III are as follows; 1,4,5,8-tetramethoxyshikonin (shown in Figure 5) and 1,4,5,8-tetramethoxyalkannin (shown in Figure 6) with a high optical purity are used as starting materials (for the synthetic methods of 1,4,5,8-tetramethoxyshikonin and 1,4,5,8-tetramethoxyalkannin, reference is made to CN102399139A).

1,4,5,8-tetramethoxyshikonin or 1,4,5,8-tetramethoxyalkannin (i.e., (R)- or (S)-4-methyl-1-(1,4,5,8-tetramethxynaphthalen-2-yl)pent-3-en-1-ol) was dissolved in dry CH₂Cl₂, 1.2-2 equivalent of carboxylic acid (all the equivalent referred in the following is molar equivalent), 1.5-5 equivalent of DCC, and DMAP (in an amount of a catalyst) were added, and the mixture was stirred for 2-12 h at room temperature. After the completion of the reaction as indicated by thin-layer chromatography (TLC), the mixture was filtered, removing the precipitate produced by the reaction. The filtrate was cooled to 0-5 °C, an aqueous solution (10 mL) of 5-10 equivalent of ceric ammonium nitrate was added into the filtrate under stirring, and the mixture was further stirred for 5-15 minutes; after the completion of the reaction as indicated by TLC, the product was extracted with DCM, the organic phase was kept and dried by anhydrous sodium sulfate; after removing the sodium sulfate by filtration, the solvent is then removed by evaporation. The product was purified by column chromatography, and the yellow-colored product was collected. The obtained product was dissolved in dry ethanol; after adding 2.2-5 equivalent of hydroxylamine hydrochloride and 3-8 equivalent of pyridine, the mixture was stirred overnight; yellow solid product was produced during the reaction; after the completion of the reaction as indicated by TLC, the stirring was stopped, the mixture was then filtered, and the filter cake was recrystallized by using ethyl acetate / petroleum ether, obtaining an ester derivative of shikonin (or alkannin) with hydroxyl methylation and carbonyl oximation on the naphthazarin parent nucleus.

The specific synthesis of the compounds of series I and III are as follows:
For the synthesis of compounds I-1 and III-1, 1.5 equivalent of DCC, 1.2 equivalent of acetic acid, 5 equivalent of ceric ammonium nitrate, 2.2 equivalent of hydroxylamine hydrochloride and 3 equivalent of pyridine were used. For the synthesis of compounds I-2 and III-2, 5 equivalent of DCC, 2 equivalent of 3,3-dimethylacrylic acid, 10 equivalent of ceric ammonium nitrate, 5 equivalent of hydroxylamine hydrochloride and 8 equivalent of pyridine were used. For the synthesis of compounds I-3 and III-3, 3 equivalent of DCC, 1.5 equivalent of 3-hydroxy-3-methylbutanoic acid, 7 equivalent of ceric ammonium nitrate, 3.5 equivalent of hydroxylamine hydrochloride and 5 equivalent of pyridine were used. For the synthesis of compounds I-4-I-16 and III-4-III-16, 1.5 equivalent of DCC, 1.2 equivalent of carboxylic acid, 5 equivalent of ceric ammonium nitrate, 2.2 equivalent of hydroxylamine hydrochloride and 3 equivalent of pyridine were used.

### 2. Preparation of ether derivatives in series II and IV:

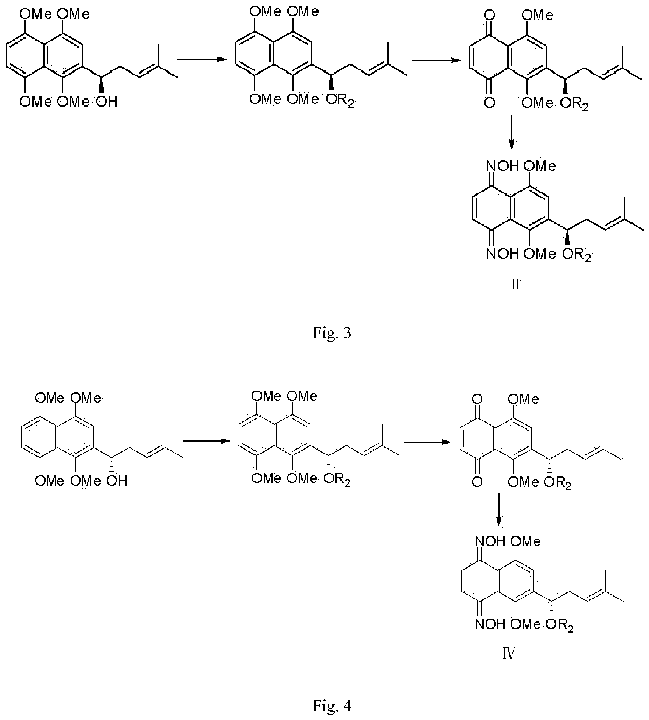

The scheme for the synthesis of ether derivatives in series II is shown in Figure 3. The scheme for the synthesis of ether derivatives in series IV is shown in Figure 4. The general synthetic method for ether derivatives in series II and IV is presented as follows; the preparation of the starting material 1,4,5,8-tetramethoxyshikonin with a high optical purity is shown in Figure 5, and the preparation of the starting material 1,4,5,8-tetramethoxyalkannin with a high optical purity is shown in Figure 6.

(R)- or (S)-4-methyl-1-(1,4,5,8-tetramethxynaphthalen-2-yl)pent-3-en-1-ol was dissolved in DMF, and the mixture was cooled down to 0 °C; 3-6 equivalent of NaH was then added into the mixture; after stirring the mixture for 30 minutes, 1.5-4 equivalent of alkyl halide was added, and the reaction mixture was stirred at room temperature for 12-24 h. After the completion of the reaction as indicated by TLC, the product was extracted by ethyl acetate; the organic solvent of the obtained organic phase was removed by evaporation, and the product was then dissolved in DCM; an aqueous solution (10 mL) of 5-10 equivalent of ceric ammonium nitrate was added into said mixture, and the obtained mixture was stirred further for 5-15 minutes. After the completion of the reaction as indicated by TLC, the product was extracted with DCM, and the obtained organic phase was kept and dried by anhydrous sodium sulfate; after removing the sodium sulfate by filtration, the solvent is then removed by evaporation. The product was purified by column chromatography, and the yellow-colored product was collected. The obtained product was dried and dissolved in dry ethanol; after adding 2.2-5 equivalent of hydroxylamine hydrochloride and 3-8 equivalent of pyridine, the mixture was stirred overnight; after the completion of the reaction as indicated by TLC, the stirring was stopped, the mixture was then filtered, and the filter cake was recrystallized by using ethyl acetate / petroleum ether, obtaining an ether derivative of shikonin (or alkannin) with hydroxyl methylation and carbonyl oximation on the naphthazarin parent nucleus.

For the synthesis of compound II-1, 6 equivalent of ceric ammonium nitrate, 3 equivalent of hydroxylamine hydrochloride and 5 equivalent of pyridine were used. For the synthesis of compound II-2, 6 equivalent of NaH, 4 equivalent of iodomethane, 10 equivalent of ceric ammonium nitrate, 5 equivalent of hydroxylamine hydrochloride and 8 equivalent of pyridine were used. For the synthesis of compound II-3, 4 equivalent of NaH, 2 equivalent of bromoethane, 5 equivalent of ceric ammonium nitrate, 2.2 equivalent of hydroxylamine hydrochloride and 4.5 equivalent of pyridine were used. For the synthesis of compound II-4, 3 equivalent of NaH, 1.5 equivalent of 1-bromo-3-methylbutane, 8 equivalent of ceric ammonium nitrate, 4 equivalent of hydroxylamine hydrochloride and 6 equivalent of pyridine were used.

For the synthesis of compound IV-1, 6 equivalent of ceric ammonium nitrate, 3 equivalent of hydroxylamine hydrochloride and 5 equivalent of pyridine were used. For the synthesis of compound IV-2, 6 equivalent of NaH, 4 equivalent of iodomethane, 10 equivalent of ceric ammonium nitrate, 5 equivalent of hydroxylamine hydrochloride and 8 equivalent of pyridine were used. For the synthesis of compound IV-3, 4 equivalent of NaH, 2 equivalent of bromoethane, 5 equivalent of ceric ammonium nitrate, 2.2 equivalent of hydroxylamine hydrochloride and 4 equivalent of pyridine were used. For the synthesis of compound IV-4, 3 equivalent of NaH, 1.5 equivalent of 1-bromo-3-methylbutane, 7.5 equivalent of ceric ammonium nitrate, 7.5 equivalent of hydroxylamine hydrochloride and 8 equivalent of pyridine were used.

### 3. Spectral characterization of the compounds of series I-IV

(*R*)-1-((5*E*,8*E*)-5,8-bis(hydroxyimino)-1,4-dimethoxy-5,8-dihydronaphthalen-2-yl)-4-methylpent-3-en-1-yl acetate (I-1): yield 84%, ¹H NMR (300 MHz, DMSO): *δ* = 12.06 (s, 2 H, H_{NOH}), 7.36 (s, 2 H, H_{Quin}), 7.02 (s, 1 H, H_{Ar}), 6.00 (t, *J* = 6.3 Hz, 1 H, ArCH-), 5.10 (t, *J* = 6.3 Hz, 1 H, -CH=), 3.78 (s, 3 H, ArOCH₃), 3.62 (s, 3 H, ArOCH₃), 2.47 (d, *J* = 1.5 Hz, 2 H, - CH₂-), 2.06 (s, 3 H, -COCH₃), 1.62 (s, 3 H, -CH₃), 1.52 (s, 3 H, -CH₃). ESI-MS m/z 411 [M + Na]⁺.

The yield and ¹H NMR of (*S*)-1-((5*E*,8*E*)-5,8-bis(hydroxyimino)-1,4-dimethoxy-5,8-dihydronaphthalen-2-yl)-4-methylpent-3-en-1-yl acetate (III-1) were the same as 1-1.

(*R*)-1-((5*E*,8*E*)-5,8-bis(hydroxyimino)-1,4-dimethoxy-5,8-dihydronaphthalen-2-yl)-4-methylpent-3-en-1-yl 3-methylbut-2-enoate (I-2): yield 79%, ¹H NMR (300 MHz, DMSO): *δ* = 12.05 (s, 2 H, H_{NOH}), 7.35 (s, 2 H, H_{Quin}), 6.99 (s, 1 H, H_{Ar}), 6.03 (t, *J* = 6.2 Hz, 1 H, ArCH-), 5.77 (s, 1 H, -COCH=C-), 5.09 (s, 1 H, -CH₂CH=C-), 3.73 (s, 3 H, ArOCH₃), 3.63 (s, 3 H, ArOCH₃), 2.47(t, *J* = 1.8 Hz, 2 H, -CH₂-), 2.07 (s, 3 H, -CH=CCH₃), 1.85 (s, 3 H, - CH=CCH₃), 1.59 (s, 3 H, -CH₃), 1.51 (s, 3 H, -CH₃). ESI-MS m/z 451 [M + Na]⁺.

The yield and ¹H NMR of (*S*)-1-((5*E*,8*E*)-5,8-bis(hydroxyimino)-1,4-dimethoxy-5,8-dihydro naphthalen-2-yl)-4-methylpent-3-en-1-yl 3-methylbut-2-enoate (III-2) were the same as I-2.

(*R*)-1-((5*E*,8*E*)-5,8-bis(hydroxyimino)-1,4-dimethoxy-5,8-dihydronaphthalen-2-yl)-4-methylpent-3-en-1-yl 3-hydroxy-3-methylbutanoate (I-3): yield 78%, ¹H NMR (300 MHz, DMSO): *δ* = 12.08 (s, 2 H, H_{NOH}), 7.38 (s, 2 H, H_{Quin}), 7.10 (s, 1 H, H_{Ar}), 6.00 (t, *J* = 6. 6 Hz, 1 H, ArCH-), 5.18-5.14 (m, 1 H, -CH=), 4.63 (s, 1 H, -CHOH), 3.78 (s, 3 H, ArOCH₃), 3.66 (s, 3 H, ArOCH₃), 2.54-2.43 (m, 4 H, -CH₂-), 1.64 (s, 3 H, -CH=CCH₃), 1.54 (s, 3 H, - CH=CCH₃), 1.23 (s, 3 H, -CH₃), 1.18 (s, 3 H, -CH₃). ESI-MS m/z 469 [M + Na]⁺.

The yield and ¹H NMR of of (*S*)-1-((5*E*,8*E*)-5,8-bis(hydroxyimino)-1,4-dimethoxy-5,8 - dihydronaphthalen-2-yl)-4-methylpent-3-en-1-yl 3-hydroxy-3-methylbutanoate (III-3) were the same as I-3.

(*R*)-1-((5*E*,8*E*)-5,8-bis(hydroxyimino)-1,4-dimethoxy-5,8-dihydronaphthalen-2-yl)-4-methylpent-3-en-1-yl isobutyrate (I-4): yield 79%, ¹H NMR (400 MHz, DMSO): *δ* = 12.08 (s, 2 H, H_{NOH}), 7.38 (s, 2 H, H_{Quin}), 7.02 (s, 1 H, H_{Ar}), 6.01 (t, *J* = 6.6 Hz, 1 H, ArCH-), 5.14 (s, 1 H, -CH=), 3.78 (s, 3 H, ArOCH₃), 3.66 (s, 3 H, ArOCH₃), 2.67-2.50 (m, 3 H, -COCH- and =CCH₂-), 1.66 (s, 3 H, -CH=CCH₃), 1.56 (s, 3 H, -CH=CCH₃), 1.13 (s, 3 H, -CH₃), 1.12 (s, 3 H, -CH₃). ESI-MS m/z 417 [M + H]⁺.

The yield and ¹H NMR of (*S*) -1-((5*E*,8*E*)-5,8-bis(hydroxyimino)-1,4-dimethoxy-5,8-dihydro naphthalen-2-yl)-4-methylpent-3-en-1-yl isobutyrate (III-4) were the same as I-4.

(*R*)-1-((5*E*,8*E*)-5,8-bis(hydroxyimino)-1,4-dimethoxy-5,8-dihydronaphthalen-2-yl)-4-methylpent-3-en-1-yl 3-methylbutanoate (I-5): yield 80%, ¹H NMR (300 MHz, DMSO): *δ* = 12.07 (s, 2 H, H_{NOH}), 7.37 (s, 2 H, H_{Quin}), 7.02 (s, 1 H, H_{Ar}), 6.04 (t, *J* = 7.2 Hz, 1 H, ArCH-), 5.14 (d, *J* = 6.0 Hz, 1 H, -CH=), 3.77 (s, 3 H, ArOCH₃), 3.65 (s, 3 H, ArOCH₃), 2.48 (s, 2 H, - CH₂-), 2.25 (d, *J* = 7.2 Hz, 2 H, -CH₂-), 2.0 (m, 1 H, -CH-), 1.62 (s, 3 H, -CH=CCH₃), 1.54 (s, 3 H, -CH=CCH₃), 0.89 (s, 3 H, -CH₃), 0.87 (s, 3 H, -CH₃). ESI-MS m/z 453 [M + Na]⁺.

The yield and ¹H NMR of (*S*)-1-((5*E*,8*E*)-5,8-bis(hydroxyimino)-1,4-dimethoxy-5,8-dihydronaphthalen-2-yl)-4-methylpent-3-en-1-yl 3-methylbutanoate (III-5) were the same as I-5.

(*R*)-1-((5*E*,8*E*)-5,8-bis(hydroxyimino)-1,4-dimethoxy-5,8-dihydronaphthalen-2-yl)-4-methylpent-3-en-1-yl propionate (I -6): yield 86%, ¹H NMR (400 MHz, DMSO): *δ* = 12.08 (s, 2 H, H_{NOH}), 7.38 (s, 2 H, H_{Quin}), 7.03 (s, 1 H, H_{Ar}), 6.04 (t, *J* = 6.0 Hz, 1 H, ArCH-), 5.13 (t, *J* = 6.4 Hz, 1 H, -CH=), 3.79 (s, 3 H, ArOCH₃), 3.66 (s, 3 H, ArOCH₃), 2.50-2.37 (m, 4 H, - CH₂-), 1.64 (s, 3 H, -CH=CCH₃), 1.55 (s, 3 H, -CH=CCH₃), 1.05 (t, *J* = 7.2 Hz, 3H, -CH₃). ESI-MS m/z 403 [M + H]⁺.

The yield and ¹H NMR of (*S*)-1-((5*E*,8*E*)-5,8-bis(hydroxyimino)-1,4-dimethoxy-5,8-dihydro naphthalen-2-yl)-4-methylpent-3-en-1-yl propionate (III-6) were the same as I-6.

(*R*)-1-((5*E*,8*E*)-5,8-bis(hydroxyimino)-1,4-dimethoxy-5,8-dihydronaphthalen-2-yl)-4-methylpent-3-en-1-yl methacrylate (I-7): yield 78%, ¹H NMR (400 MHz, DMSO): *δ* = 12.09 (s, 2 H, H_{NOH}), 7.39 (s, 2 H, H_{Quin}), 7.06 (s, 1 H, H_{Ar}), 6.16 (s, 1 H, -C=CH₂), 6.10 (dd, *J* = 7.2, 5.6 Hz, 1 H, C=CH₂), 5.74 (s, 1 H, ArCH-), 5.14 (t, 1H, *J* = 6.8 Hz, -CH₂C=), 3.77 (s, 3 H, ArOCH₃), 3.67 (s, 3 H, ArOCH₃), 2.62-2.53 (m, 2 H, -CH₂-), 1.92 (s, 3H, -CH₂=CCH₃), 1.64 (s, 3H, -CH₃), 1.56 (s, 3 H, -CH₃). ESI-MS m/z 415 [M + H]⁺.

The yield and ¹H NMR of (*S*)-1-((5*E*,8*E*)-5,S-bis(hydroxyimino)-1,4-dimethoxy-5,8-dihydronaphthalen-2-yl)-4-methylpent-3-en-1-yl methacrylate (III-7) were the same as I-7.

(*R*)-1-((5*E*,8*E*)-5,8-bis(hydroxyimino)-1,4-dimethoxy-5,8-dihydronaphthalen-2-yl)-4-methylpent-3-en-1-yl benzoate (I-8): yield 79%, ¹H NMR (400 MHz, DMSO): *δ* = 12.10 (s, 2 H, H_{NOH}), 8.06 (d, *J* = 6.8 Hz, 2 H, H_{Quin}), 7.70-7.39 (m, 5 H, H_{Ar}), 7.19 (s, 1 H, H_{Ar}), 6.30 (t, *J* = 6.0 Hz, 1 H, ArCH-), 5.20 (s, 1 H, =CH-), 3.75 (s, 3 H, ArOCH₃), 3.71 (s, 3 H, ArOCH₃), 2.72-2.63 (m, 2 H, -CH₂-), 1.64 (s, 3 H, -CH₃), 1.59 (s, 3 H, -CH₃). ESI-MS m/z 451 [M + H]⁺.

The yield and ¹H NMR of (*S*)-1-((5*E*,8*E*)-5,8-bis(hydroxyimino)-1,4-dimethoxy-5,8-dihydro naphthalen-2-yl)-4-methylpent-3-en-1-yl benzoate (III-8) were the same as I-8.

(*R*)-1-((5*E*,8*E*)-5,8-bis(hydroxyimino)-1,4-dimethoxy-5,8-dihydronaphthalen-2-yl)-4-methylpent-3-en-1-yl 2-fluorobenzoate (I-9): yield 77%, ¹H NMR (400 MHz, DMSO): *δ* = 12.10 (s, 2 H, H_{NOH}), 7.96 (dd, *J* = 7.6, 6.4 Hz, 1 H, H_{Ar}), 7.71-7.68 (m, 1 H, H_{Ar}), 7.41-7.35 (m, 4 H, H_{Ar} and H_{Quin}), 7.18 (s, 1 H, ArH), 6.31 (t, *J* = 5.6 Hz, 1 H, ArCH-), 5.20 (t, *J* = 6.8 Hz, 1 H, =CH-), 3.76 (s, 3 H, ArOCH₃), 3.71 (s, 3 H, ArOCH₃), 2.69-2.60 (m, 2 H, -CH₂-), 1.64 (s, 3 H, -CH₃), 1.55 (s, 3 H, -CH₃). ESI-MS m/z 469 [M + H]⁺.

The yield and ¹H NMR of (*S*)-1-((5*E*,8*E*)-5,8-bis(hydroxyimino)-1,4-dimethoxy-5,8-dihydronaphthalen-2-yl)-4-methylpent-3-en-1-yl 2-fluorobenzoate (III-9) were the same as I-9.

(*R*)-1-((5*E*,8*E*)-5,8-bis(hydroxyimino)-1,4-dimethoxy-5,8-dihydronaphthalen-2-yl)-4-methylpent-3-en-1-yl 4-fluorobenzoate(I-10): yield 79%, ¹H NMR (400 MHz, DMSO): *δ* = 12.10 (s, 2 H, H_{NOH}), 8.12 (dd, *J* = 8.4, 5.6 Hz, 2 H, H_{Quin}), 7.39-7.35 (m, 4 H, H_{Ar}), 7.19 (s, 1 H, H_{Ar}), 6.31-6.28 (m, 1 H, ArCH-), 5.20 (t, *J* = 2.0 Hz, 1 H, =CH-), 3.76 (s, 3 H, ArOCH₃), 3.70 (s, 3 H, ArOCH₃), 2.74-2.61 (m, 2 H, -CH₂-), 1.63 (s, 3 H, -CH₃), 1.58 (s, 3 H, -CH₃). ESI-MS m/z 469 [M + H]⁺.

The yield and ¹H NMR of (*S*)-1-((5*E*,8*E*)-5,8-bis(hydroxyimino)-1,4-dimethoxy-5,8-dihydro naphthalen-2-yl)-4-methylpent-3-en-1-yl 4-fluorobenzoate(III-10) were the same as I-10.

(*R*)-1-((5*E*,8*E*)-5,8-bis(hydroxyimino)-1,4-dimethoxy-5,8-dihydronaphthalen-2-yl)-4-methylpent-3-en-1-yl 2-chlorobenzoate (I-11): yield 78%, ¹H NMR (400 MHz, DMSO): *δ* = 12.11 (s, 2 H, H_{NOH}), 7.87 (d, *J* = 7.6 Hz, 1 H, H_{Ar}), 7.60 (d, *J* = 3.6 Hz, 2 H, H_{Ar}), 7.52-7.48 (m, 1 H, H_{Ar}), 7.40 (s, 2 H, H_{Quin}), 7.18 (s, 1 H, H_{Ar}), 6.30 (t, *J* = 5.6 Hz, 1 H, ArCH-), 5.21 (s, 1 H, =CH-), 3.77 (s, 3 H, ArOCH₃), 3.71 (s, 3 H, ArOCH₃), 2.69-2.63 (m, 2 H, -CH₂-), 1.65 (s, 3 H, -CH₃), 1.56 (s, 3 H, -CH₃). ESI-MS m/z 485 [M + H]⁺.

The yield and ¹H NMR of (*S*)-1-((5*E*,8*E*)-5,8-bis(hydroxyimino)-1,4-dimethoxy-5,8-dihydro naphthalen-2-yl)-4-methylpent-3-en-1-yl 2-chlorobenzoate (III-11) were the same as 1-11.

(*R*)-1-((5*E*,8*E*)-5,8-bis(hydroxyimino)-1,4-dimethoxy-5,8-dihydronaphthalen-2-yl)-4-methylpent-3-en-1-yl 4-chlorobenzoate (I-12): yield 81%, ¹H NMR (400 MHz, DMSO): *δ* = 12.10 (s, 2 H, H_{NOH}), 8.07 (d, *J* = 8.4 Hz, 2 H, H_{Ar}), 7.62 (d, *J* = 8.4 Hz, 2 H, H_{Ar}), 7.39 (s, 2 H, H_{Quin}), 7.18 (s, 1 H, H_{Ar}), 6.30 (t, *J* = 5.6 Hz, 1 H, ArCH-), 5.19 (s, 1 H, -CH=), 3.76 (s, 3 H, ArOCH₃), 3.70 (s, 3 H, ArOCH₃), 2.72-2.63 (m, 2 H, -CH₂-), 1.64 (s, 3 H, -CH₃), 1.59 (s, 3 H, -CH₃). ESI-MS m/z 485 [M + H]⁺.

The yield and ¹H NMR of (*S*)-1-((5*E*,8*E*)-5,8-bis(hydroxyimino)-1,4-dimethoxy-5,8-dihydro naphthalen-2-yl)-4-methylpent-3-en-1-yl 4-chlorobenzoate (III-12) were the same as I-12.

(*R*)-1-((5*E*,8*E*)-5,8-bis(hydroxyimino)-1,4-dimethoxy-5,8-dihydronaphthalen-2-yl)-4-methylpent-3-en-1-yl 4-methoxybenzoate (I-13) : yield 85%, ¹H NMR (400 MHz, DMSO): *δ* = 12.10 (s, 2 H, H_{NOH}), 8.01 (d, *J* = 4.8 Hz, 2 H, H_{Ar}), 7.39 (s, 2 H, H_{Quin}), 7.07 (d, *J* = 8.8 Hz, 2 H, H_{Ar}), 6.26 (t, *J* = 5.6 Hz, 1 H, ArCH-), 5.20 (s, 1 H, =CH-), 3.84 (s, 3 H, ArOCH₃), 3.74 (s, 3 H, ArOCH₃), 3.70 (s, 3 H, ArOCH₃), 2.70-2.59 (m, 2 H, -CH₂-), 1.64 (s, 3 H, -CH₃), 1.58 (s, 3 H, -CH₃). ESI-MS m/z 481 [M + H]⁺.

The yield and ¹H NMR of (*S*)-1-((5*E*,8*E*)-5,8-bis(hydroxyimino)-1,4-dimethoxy-5,8-dihydronaphthalen-2-yl)-4-methylpent-3-en-1-yl 4-methoxybenzoate (III-13) were the same as I-13.

(*R*)-1-((5*E*,8*E*)-5,8-bis(hydroxyimino)-1,4-dimethoxy-5,8-dihydronaphthalen-2-yl)-4-methylpent-3-en-1-yl 4-nitrobenzoate (I-14): yield 82%, ¹H NMR (400 MHz, DMSO): *δ* = 12.11 (s, 2 H, H_{NOH}), 8.12 (d, *J* = 24.8 Hz, 4 H, H_{Ar}), 7. 39 (s, 2 H, H_{Quin}), 7.21 (s, 1 H, H_{Ar}), 6.34 (s, 1 H, ArCH-), 5.20 (s, 1 H, =CH-), 3.77 (s, 3 H, ArOCH₃), 3.71 (s, 3 H, ArOCH₃), 2.75-2.68 (m, 2 H, -CH₂-), 1.63 (s, 3 H, -CH₃), 1.59 (s, 3 H, -CH₃). ESI-MS m/z 496 [M + H]⁺.

The yield and ¹H NMR of (*S*)-1-((5*E*,8*E*)-5,8-bis(hydroxyimino)-1,4-dimethoxy-5,8-dihydronaphthalen-2-yl)-4-methylpent-3-en-1-yl 4-nitrobenzoate (III-14) were the same as I-14.

(*R*)-1-((5*E*,8*E*)-5,8-bis(hydroxyimino)-1,4-dimethoxy-5,8-dihydronaphthalen-2-yl)-4-methylpent-3-en-1-yl 2-picolinate (I-15): yield 83%, ¹H NMR (400 MHz, DMSO): *δ* = 12.11 (s, 2 H, H_{NOH}), 8.76 (d, *J* = 4.4 Hz, 1 H, H_{Py}), 8.13 (d, *J* = 8.0 Hz, 1 H, H_{Py}), 8.04-8.00 (m, 1 H, H_{Py}), 7.68-7.65 (m, 1 H, H_{Py}), 7.39 (s, 2 H, H_{Quin}), 7.22 (s, 1 H, H_{Ar}), 6.32 (t, *J* = 6.0 Hz, 1 H, ArCH-), 5.20 (t, *J* = 6.8 Hz, 1 H, =CH-), 3.75 (s, 3 H, ArOCH₃), 3.71 (s, 3 H, ArOCH₃), 2.72-2.67 (m, 2 H, -CH₂-), 1.63 (s, 3 H, -CH₃), 1.59 (s, 3 H, -CH₃). ESI-MS m/z 474 [M + Na]⁺.

The yield and ¹H NMR of (*S*)-1-((5*E*,8*E*)-5,8-bis(hydroxyimino)-1,4-dimethoxy-5,8-dihydro naphthalen-2-yl)-4-methylpent-3-en-1-yl 2-picolinate (III-15) were the same as I-15.

(*R*)-1-((5*E*,8*E*)-5,8-bis(hydroxyimino)-1,4-dimethoxy-5,8-dihydronaphthalen-2-yl)-4-methylpent-3-en-1-yl thiophene-2-carboxylate (I-16): yield 80%, ¹H NMR (400 MHz, DMSO): *δ* = 12.11 (s, 2 H, H_{NOH}), 7.97 (d, *J* = 2.8 Hz, 1 H, H_{Thiophene}), 7.90 (d, *J* = 2.4 Hz, 1 H, H_{Thiophene}), 7.39 (s, 2 H, H_{Quin}), 7.24 (t, *J* = 4.4 Hz, 1 H, H_{Thiophene}), 7.15 (s, 1 H, H_{Ar}), 6.26-6.23 (m, 1 H, ArCH-), 5.18 (t, *J* = 6.0 Hz, 1 H, =CH-), 3.76 (s, 3 H, ArOCH₃), 3.69 (s, 3 H, ArOCH₃), 2.71-2.57 (m, 2 H, -CH₂-), 1.64 (s, 3 H, -CH₃), 1.59 (s, 3 H, -CH₃). ESI-MS m/z 457 [M + H]⁺.

The yield and ¹H NMR of (*S*)-1-((5*E*,8*E*)-5,8-bis(hydroxyimino)-1,4-dimethoxy-5,8-dihydronaphthalen-2-yl)-4-methylpent-3-en-1-yl thiophene-2-carboxylate (III-16) were the same as I-16.

(1*E*,4E)-6-((*R*)-1-hydroxy-4-methylpent-3-en-1-yl)-5,8-dimethoxynaphthalene-1,4-dione dioxime (II-1): yield 87%, ¹H NMR(300 MHz, DMSO): *δ* = 12.00 (s, 2 H, H_{NOH}), 7.37 (s, 2 H, H_{Quin}), 7.24 (s, 1 H, H_{Ar}), 5.24 (s, 1 H, =CH-), 4.92 (dd, *J* = 7.5, 4.8 Hz, 1 H, ArCH-), 3.78 (s, 3 H, ArOCH₃), 3.58 (s, 3 H, -OCH₃), 2.42-2.19 (m, 2 H, -CH₂-), 1.64 (s, 3 H, -CH₃), 1.52 (s, 3 H, -CH₃). ESI-MS m/z 347 [M + H]⁺.

The yield and ¹H NMR of (1*E*,4*E*)-6-((*S*)-1-hydroxy-4-methylpent-3-en-1-yl)-5,8-dimethoxy naphthalene-1,4-dione dioxime(IV-1) were the same as II-1.

(1*E*,4*E*)-5,8-dimethoxy-6-((*R*)-1-methoxy-4-methylpent-3-en-1yl)naphthalene-1,4-dione dioxime(II -2): yield 88%, ¹H NMR(300 MHz, DMSO): *δ* = 12.04 (s, 2 H, H_{NOH}), 7.39 (s, 2 H, H_{Quin}), 7.04 (s, 1 H, H_{Ar}), 5.19 (t, *J* = 6.9 Hz, 1 H, -CH=), 4.59 (t, *J* = 6.3 Hz, 1 H, ArCH-), 3.78 (s, 3 H, ArOCH₃), 3.59 (s, 3 H, ArOCH₃), 3.17 (s, 3 H, -OCH₃), 2.35 (t, *J* = 6.3 Hz, 2 H, -CH₂-), 1.63 (s, 3 H, -CH₃), 1.49 (s, 3 H, -CH₃). ESI-MS m/z 361 [M + H]⁺.

The yield and ¹H NMR of (1*E*,4*E*)-5,8-dimethoxy-6-((*S*)-1-methoxy-4-methylpent-3-en-1-yl) naphthalene-1,4-dione dioxime (IV-2) were the same as II-2.

(1*E*,4*E*)-6-((*R*)-1-ethoxy-4-methylpent-3-en-1-yl)-5,8-dimethoxynaphthalene-1,4-dione dioxime (II-3): yield 91%, ¹H NMR (400 MHz, DMSO): *δ* = 12.02 (s, 2 H, H_{NOH}), 7.38 (s, 2 H, H_{Quin}), 7.09 (s, 1 H, H_{Ar}), 5.20 (t, *J* = 5.6 Hz, 1 H, =CH-), 4.68 (t, *J* = 6.8 Hz, 1 H, ArCH-), 3.78 (s, 3 H, ArOCH₃), 3.59 (s, 3 H, ArOCH₃), 3.37-3.32 (m, 2 H, -OCH₂CH₃), 2.36-2.35 (m, 2 H, =CHCH₂-), 1.64 (s, 3 H, =CCH₃), 1.50 (s, 3 H, =CCH₃), 1.20 (t, *J* = 6.8 Hz, 3 H, -CH₃). ESI-MS m/z 375 [M + H]⁺.

The yield and ¹H NMR of (1*E*,4*E*)-6-((*S*)-1-ethoxy-4-methylpent-3-en-1-yl)-5,8-dimethoxy naphthalene-1,4-dione dioxime (IV-3) were the same as II-3.

(1*E*,4*E*)-6-((*R*)-1-(isopenloxy)-4-methylpent-3-en-1-yl)-5,8-dimethoxynaphthalene-1,4-dione dioxime (II-4): yield 81%, ¹H NMR (400 MHz, DMSO): *δ* = 12.02 (s, 2 H, H_{NOH}), 7.38 (d, *J* = 2.8 Hz, 2 H, H_{Quin}), 7.08 (s, 1 H, H_{Ar}), 5.21 (t, *J* = 6.8 Hz, 1 H, ArCH-), 4.65 (t, *J* = 6.8 Hz, 1 H, =CH-), 3.78 (s, 3 H, ArOCH₃), 3.60 (s, 3 H, ArOCH₃), 3.31 (t, *J* = 6.0 Hz, 2 H, -CH₂O-), 2.35-2.32 (m, 2 H, =CHCH₂-), 1.75-1.68 (m, 1 H, -CH(CH₃)₂), 1.64 (s, 3 H, =CCH₃), 1.51 (s, 3 H, =CCH₃), 1.44-1.38 (m, 2 H, -CH₂CH(CH₃)₂), 0.87 (d, *J* = 6.8 Hz, 3H, -CH₃), 0.82 (d, *J* = 6.8 Hz, 3 H, -CH₃). ESI-MS m/z 417 [M + H]⁺.

The yield and ¹H NMR of (1*E*,4*E*)-6-((*S*)-1-(isopentoxy)-4-methylpent-3-en-1-yl)-5,8-dimethoxynaphthalene-1,4-dione dioxime (IV-4) were the same as II-4.

### Embodiment 2

The series of the prepared ester or ether derivatives of shikonin with hydroxyl methylation and carbonyl oximation on the naphthazarin parent nucleus, and the ester or ether derivatives of alkannin with hydroxyl methylation and carbonyl oximation on the naphthazarin parent nucleus in Embodiment 1 were evaluated on DU145 (prostate cancer), MCF-7 (breast cancer), and K562 (leukemia) cells with respect to in vitro cytotoxicities. The antitumor potential of the compounds was displayed as IC₅₀ values that were calculated according to the equation: Inhibitory rate = (Average 0D value of comparative example - Average 0D value of the inventive example) / Average 0D value of comparative example, using shikonin (SK) as the comparative example; The results are summarized in Table 1.

**Table 1 In vitro inhibitory activity of the derivatives of shikonin with hydroxyl methylation and carbonyl oximation on the naphthazarin parent nucleus and the derivatives of alkannin with hydroxyl methylation and carbonyl oximation on the naphthazarin parent nucleus on DU145, MCF-7 and K562 cells**

| Compds. | R (R₁ or R₂) | IC₅₀(DU145) | IC₅₀(MCF-7) | IC₅₀(K562) |
|---|---|---|---|---|
| I-1 | methyl | 25.1 | 20.9 | 9.3 |
| I-2 | 3-methylbut-2-enyl | 11.3 | 6.0 | 1.4 |
| I-3 | 3-hydroxyisobutyl | 28.9 | 26 | 9.5 |
| I-4 | isopropyl | 10.2 | 4.9 | 3.3 |
| I-5 | isobutyl | 27.7 | 16.7 | 2.1 |
| I-6 | ethyl | 12.3 | 7.1 | 5.4 |
| I-7 | 1-methylvinyl | 18.0 | 4.9 | 3.1 |
| I-8 | phenyl | 19.4 | 4.5 | 1.9 |
| I-9 | 2-fluorophenyl | 19.9 | 7.7 | 1.7 |
| I-10 | 4-fluorophenyl | 18.4 | 3.6 | 1.5 |
| I-11 | 2-chlorophenyl | 18.0 | 5.8 | 2.9 |
| I-12 | 4-chlorophenyl | 16.8 | 6.6 | 2.1 |
| I-13 | 4-methoxyphenyl | 16.3 | 6.3 | 2.5 |
| I-14 | 4-nitrophenyl | 19.7 | 3.9 | 2.6 |
| I-15 | pyridin-2-yl | 12.1 | 7.6 | 3.8 |
| I-16 | thiophen-2-yl | 16.9 | 5.7 | 2.0 |
| II-1 | hydrogen | 72.4 | 57.4 | 42.7 |
| II-2 | methyl | 32.4 | 30.2 | 21.1 |
| II-3 | ethyl | 17.1 | 15.7 | 17.1 |
| II-4 | isopentyl | 26.3 | 16.2 | 12.4 |
| III-1 | methyl | 17.1 | 8.1 | 5.3 |
| III-2 | 3-methylbut-2-enyl | 18.1 | 1.6 | 0.7 |
| III-3 | 3-hydroxyisobutyl | 32.8 | 8.2 | 4.7 |
| III-4 | isopropyl | 18.0 | 3.7 | 3.0 |
| III-5 | isobutyl | 19.3 | 7.5 | 0.7 |
| III-6 | ethyl | 20.6 | 3.1 | 3.7 |
| III-7 | 1-methylvinyl | 19.5 | 2.4 | 2.6 |
| III-8 | phenyl | 16.9 | 2.8 | 1.6 |
| III-9 | 2-fluorophenyl | 14.3 | 1.8 | 1.5 |
| III-10 | 4-fluorophenyl | 17.0 | 1.5 | 1.3 |
| III-11 | 2-chlorophenyl | 16.8 | 1.7 | 1.3 |
| III-12 | 4-chlorophenyl | 21.4 | 2.4 | 1.3 |
| III-13 | 4-methoxyphenyl | 16.9 | 1.9 | 2.1 |
| III-14 | 4-nitrophenyl | 12.5 | 1.2 | 1.7 |
| III-15 | pyridin-2-yl | 20.7 | 6.3 | 3.6 |
| III-16 | thiophen-2-yl | 16.1 | 1.4 | 1.8 |
| IV-1 | hydrogen | 30.2 | 21.9 | 13.8 |
| IV-2 | methyl | 21.1 | 17.3 | 14.6 |
| IV-3 | ethyl | 10.6 | 17.1 | 15.4 |
| IV-4 | isopentyl | 30.8 | 17.6 | 13.5 |

Based on the results of Table 1, the derivatives of shikonin with hydroxyl methylation and carbonyl oximation on the naphthazarin parent nucleus and the derivatives of alkannin with hydroxyl methylation and carbonyl oximation on the naphthazarin parent nucleus prepared by the present invention show good growth inhibition activities on DU145, MCF-7 and K562 cells, and they can be used for the preparation of antitumor drugs.

### Embodiment 3

This embodiment involves a series of ester or ether derivatives of a racemic mixture of shikonin and alkannin with hydroxyl methylation and carbonyl oximation on the naphthazarin parent nucleus, which have structural formula (V) or (VI): 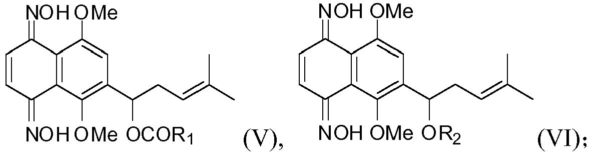 wherein R₁ and R₂ are defined in Table 2.

### 1. Preparation of the compounds (i.e., ester derivatives) in series V:

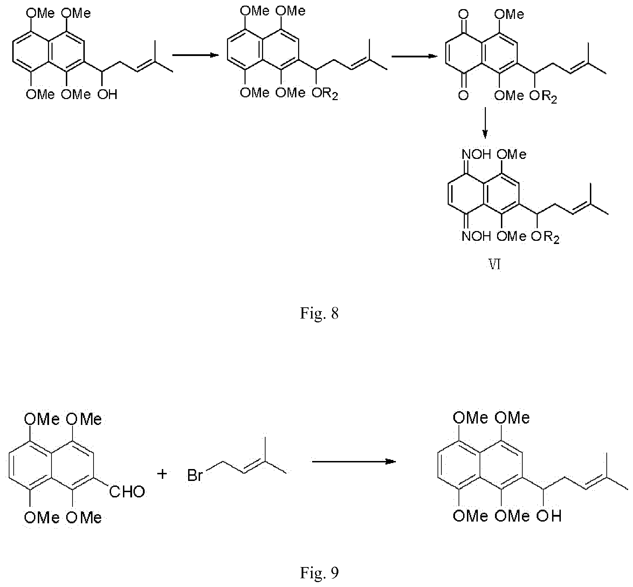

The synthetic routes of (ester derivatives in) series V are shown in Figure 7; the synthetic route of the starting material (±)-1,4,5,8-tetramethoxyshikonin is shown in Figure 9 (for the specific preparation methods, reference is made to patent ZL200510025243.1).

(±)-4-methyl-1-(1,4,5,8-tetramethxynaphthalen-2-yl)pent-3-en-1-ol was dissolved in dry CH₂Cl₂, 1.2-2 equivalent of carboxylic acid (all the equivalent referred in the following is molar equivalent), 1.5-5 equivalent of DCC, and DMAP (in an amount of a catalyst) were added, and the mixture was stirred for 2-12 h at room temperature. After the completion of the reaction as indicated by thin-layer chromatography (TLC), the mixture was filtered, removing the precipitate produced by the reaction. The filtrate was cooled to 0-5 °C, an aqueous solution (10 mL) of 3-8 equivalent of ceric ammonium nitrate was added into the filtrate under stirring, and the mixture was further stirred for 10-20 minutes; after the completion of the reaction as indicated by TLC, the product was extracted with DCM, said organic phase was kept and dried by anhydrous sodium sulfate; after removing the sodium sulfate by filtration, the solvent was then removed by evaporation. The product was purified by column chromatography, and the yellow-colored product was collected. The obtained product was dissolved in dry ethanol; after adding 2.2-4 equivalent of hydroxylamine hydrochloride and 2.5-6 equivalent of pyridine, the mixture was stirred overnight; yellow solid product was produced during the reaction; after the completion of the reaction as indicated by TLC, the stirring was stopped, the mixture was then filtered, and the filter cake was recrystallized by using ethyl acetate / petroleum ether, obtaining an ester derivative of a racemic mixture of shikonin and alkannin with hydroxyl methylation and carbonyl oximation on the naphthazarin parent nucleus (see the compounds of series V in Table 2).

For the synthesis of compound V-1, 1.5 equivalent of DCC, 1.2 equivalent of acetic acid, 3 equivalent of ceric ammonium nitrate, 2.2 equivalent of hydroxylamine hydrochloride and 2.5 equivalent of dry pyridine were used. For the synthesis of compound V-2, 5 equivalent of DCC, 2 equivalent of 3-methylbut-2-enoic acid, 8 equivalent of ceric ammonium nitrate, 4 equivalent of hydroxylamine hydrochloride and 6 equivalent of dry pyridine were used. For the synthesis of compound V-3, 3 equivalent of DCC, 1.5equivalent of 3,3-dimethylacrylic acid , 5 equivalent of ceric ammonium nitrate, 3.5 equivalent of hydroxylamine hydrochloride and 5 equivalent of dry pyridine were used. For the synthesis of compounds V-4-V-16, 1.5 equivalent of DCC, 1.2 equivalent of carboxylic acid, 3 equivalent of ceric ammonium nitrate, 2.2 equivalent of hydroxylamine hydrochloride and 2.5 equivalent of dry pyridine were used.

### 2. Preparation of the compounds (i.e., ether derivatives) in series VI:

The synthetic route of the ether compounds (i.e., ether derivatives) in series VI is shown Figure 8; the synthetic route of the starting material (±)-1,4,5,8-tetramethoxyshikonin is shown in Figure 9.

(R)- or (S)-4-methyl-1-(1,4,5,8-tetramethxynaphthalen-2-yl)pent-3-en-1-ol was dissolved in DMF, and the mixture was cooled down to 0 °C; 2-5 equivalent of NaH was then added into the mixture; after stirring the mixture for 30 minutes, 1.2-4 equivalent of alkyl halide was added, and the reaction mixture was stirred at room temperature for 12-24 h. After the completion of the reaction as indicated by TLC, the product was extracted by ethyl acetate; the organic solvent of the obtained organic phase was removed by evaporation, and the product was then dissolved in DCM; an aqueous solution (10 mL) of 3-8 equivalent of ceric ammonium nitrate was added into said mixture, and the obtained mixture was stirred further for 10-20 minutes. After the completion of the reaction as indicated by TLC, the product was extracted with DCM, and the obtained organic phase was kept and dried by anhydrous sodium sulfate; after removing the sodium sulfate by filtration, the solvent is then removed by evaporation. The product was purified by column chromatography, and the yellow-colored product was collected. The obtained product was dried and dissolved in dry ethanol; after adding 2.2-4 equivalent of hydroxylamine hydrochloride and 2.5-6 equivalent of pyridine, the mixture was stirred overnight; after the completion of the reaction as indicated by TLC, the stirring was stopped, the mixture was then filtered, and the filter cake was recrystallized by using ethyl acetate / petroleum ether, obtaining an ether derivative of a racemic mixture of shikonin and alkannin with hydroxyl methylation and carbonyl oximation on the naphthazarin parent nucleus.

For the synthesis of compound VI-1, 3 equivalent of ceric ammonium nitrate, 2.2 equivalent of hydroxylamine hydrochloride and 2.5 equivalent of dry pyridine were used. For the synthesis of compound VI-2, 5 equivalent of NaH, 4 equivalent of iodomethane, 8 equivalent of ceric ammonium nitrate, 4 equivalent of hydroxylamine hydrochloride and 6 equivalent of dry pyridine were used. For the synthesis of compound VI-3, 3 equivalent of NaH, 2.2 equivalent of bromoethane, 5 equivalent of ceric ammonium nitrate, 3 equivalent of hydroxylamine hydrochloride and 4.5 equivalent of dry pyridine were used. For the synthesis of compound VI-4, 4 equivalent of NaH, 3 equivalent of 1-bromo-3-methylbutane, 6 equivalent of ceric ammonium nitrate, 3.5 equivalent of hydroxylamine hydrochloride and 5 equivalent of dry pyridine were used.

### 3. Spectral characterization of compounds of series V and VI:

1-((5*E*,8*E*)-5,8-bis(hydroxyimino)-1,4-dimethoxy-5,8-dihydronaphthalen-2-yl)-4-methylpent-3-en-1-yl acetate (V-1): yield 86%, ¹H-NMR (300 MHz, DMSO): *δ* = 12.08 (s, 2 H, H_{N-OH}), 7.38 (s, 2 H, H_{Quin}), 7.04 (s, 1 H, H_{Ar}), 6.02 (t, *J* = 6.3 Hz, 1 H, ArCH-), 5.12 (t, *J* = 6.3 Hz, 1 H, -CH=), 3.80 (s, 3 H, ArOCH₃), 3.64 (s, 3 H, ArOCH₃), 2.49 (d, *J* = 1.5 Hz, 2 H, -CH₂-), 2.08 (s, 3 H, -COCH₃), 1.64 (s, 3 H, -CH₃), 1.54 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl 3-methylbut-2-enoate (V-2): yield 80%, ¹H-NMR (300 MHz, DMSO): *δ* = 12.06 (s, 2 H, H_{N-OH}), 7.36 (s, 2 H, H_{Quin}), 7.00 (s, 1 H, H_{Ar}), 6.04 (t, *J* = 6.2 Hz, 1 H, ArCH-), 5.78 (s, 1 H, -COCH=C-), 5.10 (s, 1 H, -CH₂CH=C), 3.74 (s, 3 H, ArOCH₃), 3.64 (s, 3 H, ArOCH₃), 2.48 (t, *J* = 1.8 Hz, 2 H, -CH₂-), 2.08 (s, 3 H, -CH=CCH₃), 1.86 (s, 3 H, -CH=CCH₃), 1.60 (s, 3 H, -CH₃), 1.52 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl 3-hydroxy-3-methylbutanoate (V-3): yield 75%, ¹H-NMR (300 MHz, DMSO): *δ* = 12.09 (s, 2 H, H_{N-OH}), 7.39 (s, 2 H, H_{Quin}), 7.11 (s, 1 H, H_{Ar}), 6.01 (t, *J* = 6. 6 Hz, 1 H, ArCH-), 5.17-5.13 (m, 1 H, -CH=), 4.64 (s, 1 H, -CHOH), 3.79 (s, 3 H, ArOCH₃), 3.67 (s, 3 H, ArOCH₃), 2.55-2.43 (m, 4 H, -CH₂-), 1.65 (s, 3 H, -CH=CCH₃), 1.55 (s, 3 H, -CH=CCH₃), 1.24 (s, 3 H, -CH₃), 1.19 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl isobutyrate (V-4): yield 72%, ¹H-NMR (300 MHz, DMSO): *δ* = 12.06 (s, 2 H, H_{N-OH}), 7.36 (s, 2 H, H_{Quin}), 7.00 (s, 1 H, H_{Ar}), 6.00 (t, *J* = 6.6 Hz, 1 H, ArCH-), 5.12 (s, 1 H, - CH=), 3.76 (s, 3 H, ArOCH₃), 3.64 (s, 3 H, ArOCH₃), 2.66-2.50 (m, 3 H, -COCH- and =CCH₂-), 1.64 (s, 3 H, -CH=CCH₃), 1.54 (s, 3 H, -CH=CCH₃), 1.11 (s, 3 H, -CH₃), 1.10 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl 3-methylbutanoate (V-5): yield 83%, ¹H-NMR (300 MHz, DMSO): *δ* = 12.07 (s, 2 H, H_{N-OH}), 7.37 (s, 2 H, H_{Quin}), 7.02 (s, 1 H, H_{Ar}), 6.04 (t, *J* = 7.2 Hz, 1 H, ArCH-), 5.14 (d, *J* = 6.0 Hz, 1 H, -CH=), 3.77 (s, 3 H, ArOCH₃), 3.65 (s, 3 H, ArOCH₃), 2.48 (s, 2 H, -CH₂-), 2.25 (d, *J* = 7.2 Hz, 2 H, -CH₂-), 2.0 (m, 1 H, -CH-), 1.62 (s, 3 H, -CH=CCH₃), 1.54 (s, 3 H, - CH=CCH₃), 0.89 (s, 3 H, -CH₃), 0.87 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl propionate (V-6): yield 76%, ¹H-NMR (400 MHz, DMSO): *δ* = 12.08 (s, 2 H, H_{N-OH}), 7.38 (s, 2 H, H_{Quin}), 7.03 (s, 1 H, H_{Ar}), 6.04 (t, *J* = 6.0 Hz, 1 H, ArCH-), 5.13 (t, *J* = 6.4 Hz, 1 H, -CH=), 3.79 (s, 3 H, ArOCH₃), 3.66 (s, 3 H, ArOCH₃), 2.50-2.37 (m, 4 H, -CH₂-), 1.64 (s, 3 H, -CH=CCH₃), 1.55 (s, 3 H, -CH=CCH₃), 1.05 (t, *J* = 7.2 Hz, 3 H, -CH₃).

1-((5*E*,8*E*)-5,8-bis(hydroxyimino)-1,4-dimethoxy-5,8-dihydronaphthalen-2-yl)-4-methylpent-3-en-1-yl methacrylate (V-7): yield 74%, ¹H-NMR (400 MHz, DMSO): *δ* = 12.09 (s, 2 H, H_{N-OH}), 7.38 (s, 2 H, H_{Quin}), 7.05 (s, 1 H, H_{Ar}), 6.15 (s, 1 H, -C=CH₂), 6.10 (dd, *J* = 7.2, 5.6 Hz, 1 H, -C=CH₂), 5.73 (s, 1 H, ArCH-), 5.12 (t, 1 H, *J* = 6.8 Hz, -CH₂C=), 3.75 (s, 3 H, ArOCH₃), 3.66 (s, 3 H, ArOCH₃), 2.61-2.54 (m, 2 H, -CH₂-), 1.91 (s, 3H, -CH₂=CCH₃), 1.63 (s, 3H, - CH₃), 1.55 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl benzoate (V-8): yield 75%, ¹H-NMR (400 MHz, DMSO): *δ* = 12.10 (s, 2 H, H_{N-OH}), 8.06 (d, *J* = 6.8 Hz, 2 H, H_{Quin}), 7.70-7.39 (m, 5 H, H_{Ar}), 7.19 (s, 1 H, H_{Ar}), 6.30 (t, *J* = 6.0 Hz, 1 H, ArCH-), 5.20 (s, 1 H, =CH-), 3.75 (s, 3 H, ArOCH₃), 3.71 (s, 3 H, ArOCH₃), 2.72-2.63 (m, 2 H, -CH₂-), 1.64 (s, 3 H, -CH₃), 1.59 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl 2-fluorobenzoate (V-9): yield 73%, ¹H-NMR (400 MHz, DMSO): *δ* = 12.10 (s, 2 H, H_{N-OH}), 7.97 (dd, *J* = 7.6, 6.4 Hz, 1 H, H_{Ar}), 7.71-7.67 (m, 1 H, H_{Ar}), 7.41-7.36 (m, 4 H, H_{Ar} and H_{Quin}), 7.19 (s, 1 H, ArH), 6.31 (t, *J* = 5.6 Hz, 1 H, ArCH-), 5.21 (t, *J* = 6.8 Hz, 1 H, =CH-), 3.78 (s, 3 H, ArOCH₃), 3.72 (s, 3 H, ArOCH₃), 2.69-2.61 (m, 2 H, -CH₂-), 1.66 (s, 3 H, - CH₃), 1.56 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl 4-fluorobenzoate (V-10): yield 80%, ¹H-NMR (400 MHz, DMSO): *δ* = 12.10 (s, 2 H, H_{N-OH}), 8.12 (dd, *J* = 8.4, 5.6 Hz, 2 H, H_{Quin}), 7.39-7.35 (m, 4 H, H_{Ar}), 7.19 (s, 1 H, H_{Ar}), 6.31-6.28 (m, 1 H, ArCH-), 5.20 (t, *J* = 2.0 Hz, 1 H, =CH-), 3.76 (s, 3 H, ArOCH₃), 3.70 (s, 3 H, ArOCH₃), 2.74-2.61 (m, 2 H, -CH₂-), 1.63 (s, 3 H, -CH₃), 1.58 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl 2-chlorobenzoate (V-11): yield 81%, ¹H-NMR (400 MHz, DMSO): *δ* = 12.11 (s, 2 H, H_{N-OH}), 7.87 (d, *J* = 7.6 Hz, 1 H, H_{Ar}), 7.60 (d, *J* = 3.6 Hz, 2 H, H_{Ar}), 7.52-7.48 (m, 1 H, H_{Ar}), 7.40 (s, 2 H, H_{Quin}), 7.18 (s, 1 H, H_{Ar}), 6.30 (t, *J* = 5.6 Hz, 1 H, ArCH-), 5.21 (s, 1 H, =CH-), 3.77 (s, 3 H, ArOCH₃), 3.71 (s, 3 H, ArOCH₃), 2.69-2.63 (m, 2 H, -CH₂-), 1.65 (s, 3 H, -CH₃), 1.56 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl 4-chlorobenzoate (V-12): yield 83%, ¹H-NMR (400 MHz, DMSO): *δ* = 12.11 (s, 2 H, H_{N-OH}), 8.08 (d, *J* = 8.4 Hz, 2 H, H_{Ar}), 7.63 (d, *J* = 8.4 Hz, 2 H, H_{Ar}), 7.40 (s, 2 H, H_{Quin}), 7.19 (s, 1 H, H_{Ar}), 6.31 (t, *J* = 5.6 Hz, 1 H, ArCH-), 5.20 (s, 1 H, -CH=), 3.77 (s, 3 H, ArOCH₃), 3.71 (s, 3 H, ArOCH₃), 2.73-2.64 (m, 2 H, -CH₂-), 1.65 (s, 3 H, -CH₃), 1.60 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl 4-methoxybenzoate (V-13): yield 86%, ¹H-NMR (400 MHz, DMSO): *δ* = 12.10 (s, 2 H, H_{NOH}), 8.01 (d, *J* = 4.8 Hz, 2 H, H_{Ar}), 7.39 (s, 2 H, H_{Quin}), 7.07 (d, *J* = 8.8 Hz, 2 H, H_{Ar}), 6.26 (t, *J* = 5.6 Hz, 1 H, ArCH-), 5.20 (s, 1 H, =CH-), 3.84 (s, 3 H, ArOCH₃), 3.74 (s, 3 H, ArOCH₃), 3.70 (s, 3 H, ArOCH₃), 2.70-2.59 (m, 2 H, -CH₂-), 1.64 (s, 3 H, -CH₃), 1.58 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl 4-nitrobenzoate (V-14): yield 82%, ¹H-NMR (400 MHz, DMSO): *δ* = 12.11 (s, 2 H, H_{N-OH}), 8.12 (d, *J* = 24.8 Hz, 4 H, H_{Ar}), 7. 39 (s, 2 H, H_{Quin}), 7.21 (s, 1 H, H_{Ar}), 6.34 (s, 1 H, ArCH-), 5.20 (s, 1 H, =CH-), 3.77 (s, 3 H, ArOCH₃), 3.71 (s, 3 H, ArOCH₃), 2.75-2.68 (m, 2 H, -CH₂-), 1.63 (s, 3 H, -CH₃), 1.59 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl 2-picolinate (V-15): yield 78%, ¹H-NMR (400 MHz, DMSO): *δ* = 12.07 (s, 2 H, H_{N-OH}), 8.73 (d, *J* = 4.4 Hz, 1 H, H_{Py}), 8.10 (d, *J* = 8.0 Hz, 1 H, H_{Py}), 8.01-8.00 (m, 1 H, H_{Py}), 7.66-7.60 (m, 1 H, H_{Py}), 7.37 (s, 2 H, H_{Quin}), 7.20 (s, 1 H, H_{Ar}), 6.30 (t, *J* = 6.0 Hz, 1 H, ArCH-), 5.18 (t, *J* = 6.8 Hz, 1 H, =CH-), 3.73 (s, 3 H, ArOCH₃), 3.69 (s, 3 H, ArOCH₃), 2.72-2.67 (m, 2 H, -CH₂-), 1.63 (s, 3 H, -CH₃), 1.59 (s, 3 H, -CH₃).

1-((5*E*,8*E*)-5,8-bis(hydroxyimino)-1,4-dimethoxy-5,8-dihydronaphthalen-2-yl)-4-methylpent-3-en-1-yl thiophene-2-carboxylate (V-16): yield 76%, ¹H-NMR (400 MHz, DMSO): *δ* = 12.12 (s, 2 H, H_{N-OH}), 7.98 (d, *J* = 2.8 Hz, 1 H, H_{Thiophene}), 7.91 (d, *J* = 2.4 Hz, 1 H, H_{Thiophene}), 7.40 (s, 2 H, H_{Quin}), 7.25 (t, *J* = 4.4 Hz, 1 H, H_{Thiophene}), 7.16 (s, 1 H, H_{Ar}), 6.25-6.21 (m, 1 H, ArCH-), 5.19 (t, *J* = 6.0 Hz, 1 H, =CH-), 3.77(s, 3 H, ArOCH₃), 3.70 (s, 3 H, ArOCH₃), 2.72-2.57 (m, 2 H, -CH₂-), 1.65 (s, 3 H, -CH₃), 1.60 (s, 3 H, -CH₃).

(1*E*,4*E*)-6-(1-hydroxy-4-methylpent-3-en-1-yl)-5,8-dimethoxynaphthalene-1,4-dione dioxime (VI-1): yield 83%, ¹H-NMR (400 MHz, DMSO): *δ* = 12.02(s, 2 H, H_{N-OH}), 7.39 (s, 2 H, H_{Quin}), 7.26 (s, 1 H, H_{Ar}), 5.27 (s, 1 H, =CH-), 4.96 (dd, *J* = 7.6, 4.8 Hz, 1 H, ArCH-), 3.80 (s, 3 H, ArOCH₃), 3.61 (s, 3 H, OCH₃), 2.44-2.22 (m, 2 H, -CH₂-), 1.66 (s, 3 H, -CH₃), 1.53 (s, 3 H, -CH₃).

(1*E*,4*E*)-6-(1-methoxy-4-methylpent-3-en-1-yl)-5,8-dimethoxynaphthalene-1,4-dione dioxime (VI-2): yield 85%, ¹H-NMR (300 MHz, DMSO): *δ* = 12.04 (s, 2 H, H_{N-OH}), 7.39 (s, 2 H, H_{Quin}), 7.04 (s, 1 H, H_{Ar}), 5.19 (t, *J* = 6.9 Hz, 1 H, -CH=), 4.59 (t, *J* = 6.3 Hz, 1 H, ArCH-), 3.78 (s, 3 H, ArOCH₃), 3.59 (s, 3 H, ArOCH₃), 3.17 (s, 3 H, -OCH₃), 2.35 (t, *J* = 6.3 Hz, 2 H, -CH₂-), 1.63 (s, 3 H, -CH₃), 1.49 (s, 3 H, -CH₃).

(1*E*,4*E*)-6-(1-ethoxy-4-methylpent-3-en-1-yl)-5,8-dimethoxynaphthalene-1,4-dione dioxime (VI-3): yield 89%, ¹H-NMR (400 MHz, DMSO): *δ* = 12.02 (s, 2 H, H_{N-OH}), 7.38 (s, 2 H, H_{Quin}), 7.09 (s, 1 H, H_{Ar}), 5.20 (t, *J* = 5.6 Hz, 1 H, =CH-), 4.68 (t, *J* = 6.8 Hz, 1 H, ArCH-), 3.78 (s, 3 H, ArOCH₃), 3.59 (s, 3 H, ArOCH₃), 3.37-3.32 (m, 2 H, -OCH₂CH₃), 2.36-2.35 (m, 2 H, =CHCH₂-), 1.64 (s, 3 H, =CCH₃), 1.50 (s, 3 H, =CCH₃), 1.20 (t, *J* = 6.8 Hz, 3 H, -CH₃).

(1*E*,4*E*)-6-(1-(isopentoxy)-4-methylpent-3-en-1-yl)-5,8-dimethoxynaphthalene-1,4-dione dioxime (VI-4): yield 77%, ¹H-NMR (400 MHz, DMSO): *δ* = 12.02 (s, 2 H, H_{NOH}), 7.38 (d, *J* = 2.8 Hz, 2 H, H_{Quin}), 7.08 (s, 1 H, H_{Ar}), 5.21 (t, *J* = 6.8 Hz, 1 H, ArCH-), 4.65 (t, *J* = 6.8 Hz, 1 H, =CH-), 3.78 (s, 3 H, ArOCH₃), 3.60 (s, 3 H, ArOCH₃), 3.31 (t, *J* = 6.0 Hz, 2 H, -CH₂O-), 2.35-2.32 (m, 2 H, =CHCH₂-), 1.75-1.68 (m, 1 H, -CH(CH₃)₂), 1.64 (s, 3 H, =CCH₃), 1.51 (s, 3 H, =CCH₃), 1.44-1.38 (m, 2 H, -CH₂CH(CH₃)₂), 0.87 (d, *J* = 6.8 Hz, 3H, -CH₃), 0.82 (d, *J* = 6.8 Hz, 3 H, -CH₃).

### Embodiment 4

The prepared ester or ether derivatives of a racemic mixture of shikonin and alkannin with hydroxyl methylation and carbonyl oximation on the naphthazarin parent nucleus in Embodiment 3 were evaluated on MCF-7 (breast cancer), K562 (leukemia) and DU145 (prostate cancer) cells respect to in vitro cytotoxicities; antitumor potential of the compounds was displayed as IC₅₀ values that were calculated according to the equation: Inhibitory rate = (Average 0D value of the comparative example - Average 0D value of the inventive example) / Average 0D value of the comparative example, using shikonin (SK) as the comparative example; the results are summarized in Table 2.

**Table 2 In vitro inhibitory activity of the derivatives of a racemic mixture of shikonin and alkannin with hydroxyl methylation and carbonyl oximation on the naphthazarin parent nucleus on DU145, MCF-7 and K562 cells**

| Compds. | R (R₁ or R₂) | IC₅₀(DU145) | IC₅₀(MCF-7) | IC₅₀(K562) |
|---|---|---|---|---|
| V-1 | methyl | 24.2 | 14.7 | 7.3 |
| V-2 | 3-methylbut-2-enyl | 10.3 | 3.6 | 1.5 |
| V-3 | 3-hydroxyisobutyl | 29.9 | 15.8 | 6.5 |
| V-4 | isopropyl | 9.3 | 4.0 | 3.3 |
| V-5 | isobutyl | 17.8 | 12.7 | 1.3 |
| V-6 | ethyl | 12.6 | 5.3 | 5.0 |
| V-7 | 1-methylvinyl | 10.3 | 3.6 | 3.2 |
| V-8 | phenyl | 19.4 | 3.5 | 1.9 |
| V-9 | 2-fluorophenyl | 20.9 | 4.7 | 1.8 |
| V-10 | 4-fluorophenyl | 18.2 | 3.3 | 1.5 |
| V-11 | 2-chlorophenyl | 18.0 | 4.0 | 2.0 |
| V-12 | 4-chlorophenyl | 15.8 | 4.6 | 1.5 |
| V-13 | 4-methoxyphenyl | 16.0 | 3.3 | 1.8 |
| V-14 | 4-nitrophenyl | 10.7 | 1.9 | 2.5 |
| V-15 | pyridin-2-yl | 12.4 | 8.8 | 3.9 |
| V-16 | thiophen-2-yl | 16.6 | 4.7 | 2.0 |
| VI-1 | hydrogen | 55.4 | 40.4 | 30.7 |
| VI-2 | methyl | 32.4 | 25.2 | 19.1 |
| VI-3 | ethyl | 17.1 | 15.7 | 17.1 |
| VI-4 | isopentyl | 26.3 | 16.2 | 15.4 |

Based on the results of Table 2, the derivatives of a racemic mixture of shikonin and alkannin with hydroxyl methylation and carbonyl oximation on the naphthazarin parent nucleus prepared by the present invention show growth inhibition activities on DU145, MCF-7 and K562 cells.

The embodiments of the present invention have been described in the above. It needs to be understood that, the present invention is not limited by the above specific embodiments, the skilled person may conduct various changes or modifications within the scope of the claims, which does not affect the substantial subject-matter of the present invention.

## Claims

1. A derivative of shikonin with hydroxyl methylation and carbonyl oximation on the naphthazarin parent nucleus, having structural formula (I) or (II): 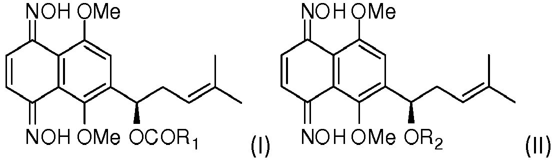 wherein R₁ is C₁-C₆ alkane, C₁-C₆ olefin, C₁-C₆ arene, or substituted arene; and R₂ is C₁-C₆ alkane, C₁-C₆ olefin, C₁-C₆ arene, or substituted arene or is H.

2. The derivative of shikonin with hydroxyl methylation and carbonyl oximation on the naphthazarin parent nucleus according to claim 1, wherein R₁ is methyl, isopropyl, isobutyl, 2-hydroxyl-2-methylpropyl, phenyl, 2-fluorophenyl, 4-fluorophenyl, 1-methylethylene, 2-clorophenyl, 4-clorophenyl, 4-methoxyphenyl, ethylene, 2-thiophenyl, 4-nitrophenyl or 2-pyridyl; R₂ is hydrogen, methyl, ethyl or isopentyl.

3. A derivative of alkannin with hydroxyl methylation and carbonyl oximation on the naphthazarin parent nucleus, having structural formula (III) or (IV): 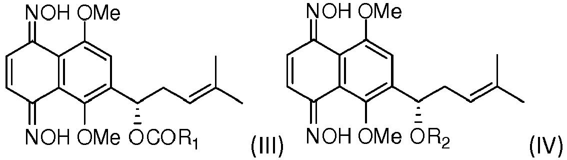 wherein R₁ is C₁-C₆ alkane, C₁-C₆ olefin, C₁-C₆ arene, or substituted arene; and R₂ is C₁-C₆ alkane, C₁-C₆ olefin, C₁-C₆ arene, or substituted arene or is H.

4. The derivative of alkannin with hydroxyl methylation and carbonyl oximation on the naphthazarin parent nucleus according to claim 3, wherein R₁ is methyl, isopropyl, isobutyl, 2-hydroxyl-2-methylpropyl, phenyl, 2-fluorophenyl, 4-fluorophenyl, 1-methylethylene, 2-clorophenyl, 4-clorophenyl, 4-methoxyphenyl, ethylene, 2-thiophenyl, 4-nitrophenyl or 2-pyridyl; R₂ is hydrogen, methyl, ethyl or isopentyl.

5. A racemic derivative of shikonin with hydroxyl methylation and carbonyl oximation on the naphthazarin parent nucleus, having structural formula (V) or (VI): 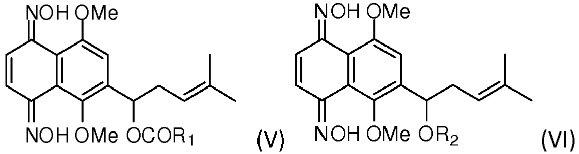 wherein R₁ is C₁-C₆ alkane, C₁-C₆ olefin, C₁-C₆ arene, or substituted arene; and R₂ is C₁-C₆ alkane, C₁-C₆ olefin, C₁-C₆ arene, or substituted arene or is H.

6. The racemic derivatives of shikonin with hydroxyl methylation and carbonyl oximation on the naphthazarin parent nucleus according to claim 5, wherein R₁ is methyl, isopropyl, isobutyl, 2-hydroxyl-2-methylpropyl, phenyl, 2-fluorophenyl, 4-fluorophenyl, 1-methylethylene, 2-clorophenyl, 4-clorophenyl, 4-methoxyphenyl, ethylene, 2-thiophenyl, 4-nitrophenyl or 2-pyridyl; R₂ is hydrogen, methyl, ethyl or isopentyl.

## Patentansprüche

1. Derivat von Shikonin mit Hydroxylmethylierung und Carbonyloximation am Naphthazarinelternkern, das die strukturelle Formel (I) oder (II) aufweist: 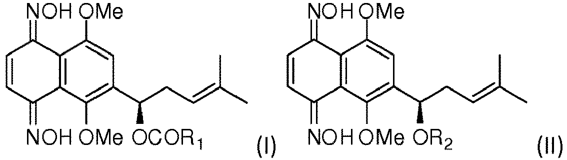 wobei R₁ C₁-C₆-Alkan, C₁-C₆-Olefin, C₁-C₆-Aren oder substituiertes Aren ist; und R₂ C₁-C₆-Alkan, C₁-C₆-Olefin, C₁-C₆-Aren oder substituiertes Aren oder H ist.

2. Derivat von Shikonin mit Hydroxylmethylierung und Carbonyloximation am Naphthazarinelternkern nach Anspruch 1, wobei R₁ Methyl, Isopropyl, Isobutyl, 2-Hydroxyl-2-methylpropyl, Phenyl, 2-Fluorphenyl, 4-Fluorphenyl, 1-Methylethylen, 2-Chlorphenyl, 4-Chlorphenyl, 4-Methoxyphenyl, Ethylen, 2-Thiophenyl, 4-Nitrophenyl oder 2-Pyridyl ist; R₂ Wasserstoff, Methyl, Ethyl oder Isopentyl ist.

3. Derivat von Alkannin mit Hydroxylmethylierung und Carbonyloximation am Naphthazarinelternkern, das die strukturelle Formel (III) oder (IV) aufweist: 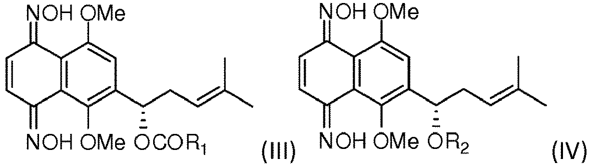 wobei R₁ C₁-C₆-Alkan, C₁-C₆-Olefin, C₁-C₆-Aren oder substituiertes Aren ist; und R₂ C₁-C₆-Alkan, C₁-C₆-Olefin, C₁-C₆-Aren oder substituiertes Aren oder H ist.

4. Derivat von Alkannin mit Hydroxylmethylierung und Carbonyloximation am Naphthazarinelternkern nach Anspruch 3, wobei R₁ Methyl, Isopropyl, Isobutyl, 2-Hydroxyl-2-methylpropyl, Phenyl, 2-Fluorphenyl, 4-Fluorphenyl, 1-Methylethylen, 2-Chlorphenyl, 4-Chlorphenyl, 4-Methoxyphenyl, Ethylen, 2-Thiophenyl, 4-Nitrophenyl oder 2-Pyridyl ist; R₂ Wasserstoff, Methyl, Ethyl oder Isopentyl ist.

5. Racemisches Derivat von Shikonin mit Hydroxylmethylierung und Carbonyloximation am Naphthazarinelternkern, das die strukturelle Formel (V) oder (V) aufweist: 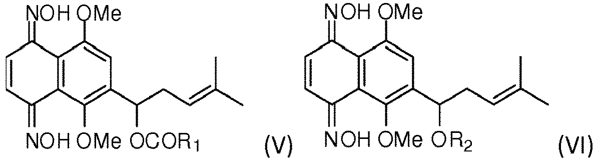 wobei R₁ C₁-C₆-Alkan, C₁-C₆-Olefin, C₁-C₆-Aren oder substituiertes Aren ist; und R₂ C₁-C₆-Alkan, C₁-C₆-Olefin, C₁-C₆-Aren oder substituiertes Aren oder H ist.

6. Racemisches Derivat von Shikonin mit Hydroxylmethylierung und Carbonyloximation am Naphthazarinelternkern nach Anspruch 5, wobei R₁ Methyl, Isopropyl, Isobutyl, 2-Hydroxyl-2-methylpropyl, Phenyl, 2-Fluorphenyl, 4-Fluorphenyl, 1-Methylethylen, 2-Chlorphenyl, 4-Chlorphenyl, 4-Methoxyphenyl, Ethylen, 2-Thiophenyl, 4-Nitrophenyl oder 2-Pyridyl ist; R₂ Wasserstoff, Methyl, Ethyl oder Isopentyl ist.

## Revendications

1. Dérivé de la shikonine avec une méthylation d'hydroxyle et une oxymation de carbonyle sur le noyau parent de la naphtazarine possédant les formules structurelles (I) ou (II) : 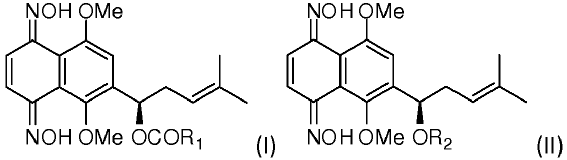 dans lesquelles R₁ représente C₁-C₆ alkane, C₁-C₆ oléfine, C₁-C₆ arène, ou arène substitué ; et R₂ représente C₁-C₆ alkane, C₁-C₆ oléfine, C₁-C₆ arène, ou arène substitué ou représente H.

2. Dérivé de la shikonine avec une méthylation d'hydroxyle et une oxymation de carbonyle sur le noyau parent de la naphtazarine selon la revendication 1, dans lequel R₁ représente méthyle, isopropyle, isobutyle, 2-hydroxyl-2-méthylpropyle, phényle, 2-fluorophényle, 4-fluorophényle, 1-méthyléthylène, 2-chlorophényle, 4-chlorophényle, 4-méthoxyphényle, éthylène, 2-thiophényle, 4-nitrophényle ou 2-pyridyle ; R₂ représente hydrogène, méthyle, éthyle ou isopentyle.

3. Dérivé de l'alkannine avec une méthylation d'hydroxyle et une oxymation de carbonyle sur le noyau parent de la naphtazarine possédant les formules structurelles (III) ou (IV) : 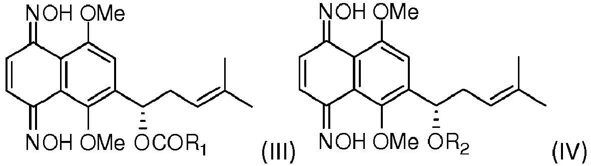 dans lesquelles R₁ représente C₁-C₆ alkane, C₁-C₆ oléfine, C₁-C₆ arène, ou arène substitué ; et R₂ représente C₁-C₆ alkane, C₁-C₆ oléfine, C₁-C₆ arène, ou arène substitué ou représente H.

4. Dérivé de l'alkannine avec une méthylation d'hydroxyle et une oxymation de carbonyle sur le noyau parent de la naphtazarine selon la revendication 3, dans lequel R1 représente méthyle, isopropyle, isobutyle, 2-hydroxyl-2-méthylpropyle, phényle, 2-fluorophényle, 4-fluorophényle, 1-méthyléthylène, 2-chlorophényle, 4-chlorophényle, 4-méthoxyphényle, éthylène, 2-thiophényle, 4-nitrophényle ou 2-pyridyle ; R2 représente hydrogène, méthyle, éthyle ou isopentyle.

5. Dérivé racémique de la shikonine avec une méthylation d'hydroxyle et une oxymation de carbonyle sur le noyau parent de la naphtazarine, possédant les formules structurelles (V) ou (VI) : 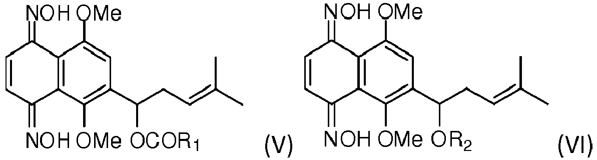 dans lesquelles R₁ représente C₁-C₆ alkane, C₁-C₆ oléfine, C₁-C₆ arène, ou arène substitué ; et R₂ représente C₁-C₆ alkane, C₁-C₆ oléfine, C₁-C₆ arène, ou arène substitué ou représente H.

6. Dérivés racémiques de la shikonine avec une méthylation d'hydroxyle et une oxymation de carbonyle sur le noyau parent de la naphtazarine selon la revendication 5, dans lesquels R₁ représente méthyle, isopropyle, isobutyle, 2-hydroxyl-2-méthylpropyle, phényle, 2-fluorophényle, 4-fluorophényle, 1-méthyléthylène, 2-chlorophényle, 4-chlorophényle, 4-méthoxyphényle, éthylène, 2-thiophényle, 4-nitrophényle ou 2-pyridyle ; R₂ représente hydrogène, méthyle, éthyle ou isopentyle.
